# EUROPÄISCHE PATENTSCHRIFT

(11) **EP 1 922 093 B1**
(45) Veröffentlichungstag und Bekanntmachung des Hinweises auf die Patenterteilung: **13.07.2011**
(21) Anmeldenummer: 06806752.9
(22) Anmeldetag: 06.09.2006
(51) Int. Cl.: A61L 27/36

(54) **NERVENIMPLANTAT**
NEURAL IMPLANT
IMPLANT NERVEUX

(30) Priorität: 06.09.2005 DE 102005042455
(43) Veröffentlichungstag der Anmeldung: 21.05.2008
(73) Patentinhaber: Medizinische Hochschule Hannover, 30625 Hannover (DE)
(72) Erfinder: VOGT, Peter, 30625 Hannover (DE); ALLMELING, Christina, 31228 Peine (DE); REIMERS, Kerstin, 30627 Hannover (DE)
(74) Vertreter: Taruttis, Stefan Georg
(86) Internationale Anmeldenummer: PCT/EP2006/066049
(87) Internationale Veröffentlichungsnummer: WO 2007/028801

(56) Entgegenhaltungen:
- EP-A1- 1 084 686
- WO-A-2005/094911
- WO-A-2006/030182
- US-A1- 2001 053 931
- ALLMELING C. ET AL.: "Use of spider silk fibres as an innovative material in a biocompatible artificial nerve conduit" JOURNAL OF CELLULAR AND MOLECULAR MEDICINE, Bd. 10, Nr. 3, 2006, Seiten 770-777, XP002442484

## Beschreibung

Die vorliegende Erfindung betrifft ein biokompatibles Nervenimplantat zur Überbrückung von Unterbrechungen oder von Fehlstellen bei Nerven, die beispielsweise durch Unfallverletzungen oder nach Operationen auftreten. Erfindungsgemäße Nervenimplantate sind geeignet, die Regeneration unterbrochener Nerven auch über Fehlstellen hinweg zu ermöglichen, so dass innerhalb kurzer Zeit eine funktionale Reizleitung auch über die von dem Nervenimplantat überbrückte Fehlstelle hinweg möglich ist.

### Stand der Technik

Bisher wird versucht, Unterbrechungen in Nerven oder Fehlstellen innerhalb von Nerven, bei denen die getrennten Nervenenden nicht ohne weiteres miteinander verbunden werden können, dadurch eine Hilfestellung zur Regeneration zu geben, dass die Fehlstelle mit einem autologen Nervenstück überbrückt wird, das beispielsweise von einem peripheren Nerv stammt.

Weiterhin ist es bekannt, periphere Nervenverletzungen dadurch zu behandeln, dass die Fehlstellen von einer eingesetzten autologen Venole oder einem Collagenröhrchen überbrückt wird.

Daneben ist es aus der EP 1523 999 A1 bekannt, endovasculäre Transplantate, so genannte Stents, durch einen Gehalt an Spinnenseide mit größerer mechanischer Festigkeit auszustatten.

Die WO 01/56626 beschreibt allein die Verwendung von Spinnenseide als Nahtmaterial, nicht jedoch als Implantat, mit dem Unterbrechungen von Nerven überbrückt werden könnten.

Die WO 2005/094911, die nach dem Prioritätstag der vorliegenden Anmeldung veröffentlicht und daher nur in Bezug auf die Neuheit relevanter Stand der Technik ist, beschreibt Verbundmaterialien zur Herstellung von Implantaten, darunter Hüllen, die zur Regeneration von Nerven dienen sollen. Die WO 2005/094911 erwähnt nicht, dass bereits reine Spinnenseide geeignet ist, Nervenzellen über Defektstellen hinweg wachsen zu lassen, sondern beschreibt eine Anzahl alternativer Verbundmaterialien, aus denen formbeständige Implantate hergestellt werden können.

Die EP 1084686 A1 beschreibt ein Nervenimplantat mit einem Röhrchen aus Gelatine oder Kollagen mit axial darin angeordneten dehydrierten Kollagenfasern, die mit Laminin beschichtet sind.

Die US 2001/0053931 A1 beschreibt einen implantierbaren Stent, der ein Röhrchen aus natürlicher oder gentechnisch gewonnener Spinnenseide aufweist.

Die WO2006/030182, die nach dem Prioritätstag der vorliegenden Anmeldung veröffentlicht ist und daher nur in Bezug auf die Neuheit relevant ist, beschreibt ein Nervenimplantat, das längs in einem Röhrchen angeordnete Spinnenseide aufweist.

Die US 2003/0100108 A1 beschreibt die Herstellung von Sehnenimplantaten auf einer Matrix, die auch Seidenfäden enthält. Bei diesen Sehnenimplantaten werden die mechanischen Eigenschaften von Seidenfäden benützt, jedoch nicht deren Eignung zur Verwendung, Nerven über Defektstellen wachsen zu lassen.

Die DE 100 53 611 A1 beschreibt ein Leitrohr, in dem Axone wachsen sollen. Dieses Leitrohr, auch als Leitschiene bezeichnet, besteht aus polymerisierten Hydroxycarbonsäuren, in dem durch Extrusion hergestellte synthetische Monofilamente angeordnet sein können.

### Aufgabe der Erfindung

Gegenüber den bekannten Transplantaten zur Überbrückung von Fehlstellen in Nerven ist es eine Aufgabe der vorliegenden Erfindung, eine Alternative zu autologen Transplantaten oder Verbundmaterialien bereitzustellen. Eine besondere Aufgabe der vorliegenden Erfindung liegt darin, ein Nervenimplantat mit verbesserten Eigenschaften bezüglich der Wiederherstellung der Reizleitung und Regeneration des unterbrochenen Nervs bereitzustellen. Eine weitere Aufgabe liegt in der Bereitstellung eines Nervenimplantats, das dabei keine autologen Nerven, und besonders bevorzugt, auch kein anderes autologes Gewebe erfordert bzw. enthält.

Eine besondere Aufgabe der vorliegenden Erfindung besteht darin, ein Nervenimplantat zur Verwendung bei der Herstellung einer pharmazeutischen Zusammensetzung bereitzustellen, mit dem Fehlstellen innerhalb von Nerven überbrückt werden können, wobei bei dessen Verwendung vorzugsweise nur eine operative Behandlung ausreicht, um die Regeneration des Nervs zur funktionalen Reizleitung zu ermöglichen und, besonders bevorzugt, zusätzlich zum Aufbau des natürlichen Nervengewebes über den Bereich der Fehlstelle.

### Allgemeine Beschreibung der Erfindung

Zur Lösung der vorgenannten Aufgaben stellt die Erfindung ein Nervenimplantat nach Anspruch 1 bereit, das Spinnenseide aufweist. Insbesondere stellt die Erfindung Spinnenseide zur Verwendung für die Produktion einer pharmazeutischen Zusammensetzung bereit, deren medizinische Indikation Defektstellen von Nerven sind, die überbrückt werden sollen.

In einer ersten Ausführungsform wird das erfindungsgemäße Nervenimplantat von Fäden von Spinnenseide gebildet, die innerhalb einer schlauchartigen Hülle angeordnet sind. Eine solche Hülle, die die Fäden der Spinnenseide aufnimmt, sind autologe Venolen, xenologe azellularisierte Venolen oder immunologisch kompatible Venolen.

In einer zweiten Ausführungsform wird das erfindungsgemäße Nervenimplantat aus fadenförmiger Spinnenseide gebildet, die um ein Trägerelement herum angeordnet ist. Als Trägerelement werden autologe oder azellularisierte Venolen, sowie streifenförmige Elemente aus der Wandung autologer oder azellularisierter Venolen, eingesetzt.

Die Ausführungsformen des erfindungsgemäßen Nervenimplantats sind geeignet, um nach chirurgischer Einfügung in eine Unterbrechung oder Fehlstelle von Nerven die funktionale Reizleitung innerhalb kurzer Zeit wieder herzustellen und die Bildung von natürlichem Nervengewebe über die Unterbrechung bzw. Fehlstelle hinweg zu bewirken. Entsprechend können die erfindungsgemäßen Ausführungsformen verwendet werden zur Herstellung eines Materials zur medizinischen Anwendung zur Behandlung von Fehlstellen von Nerven, so dass die Fehlstellen durch gerichtetes Wachsen von Nervengewebe die Fehlstelle überbrückt.

So lässt sich bei der Überbrückung von Fehlstellen in motorischen Nerven von Säugetieren über eine Distanz von 2 bis 4,5 cm oder mehr zeigen, dass die Reizleitung zumindest teilweise wieder hergestellt werden kann, und innerhalb weniger Stunden bis zu 2 bis 5 Tagen die Funktionsfähigkeit des Nervs über seine Unterbrechung bzw. Fehlstelle hinweg im wesentlichen, vorzugsweise vollständig, wieder hergestellt ist. Innerhalb eines Zeitraums von 2 bis 10 Tagen, vorzugsweise 3 bis 4 Tagen, lässt sich die Regeneration von Nervengewebe in der vom erfindungsgemäßen Nervenimplantat eingenommenen Stelle der Unterbrechung oder Fehlstelle des Nervs durch Bildung funktionalen Nervengewebes nachweisen, das vorzugsweise keine histologischen Unterschiede gegenüber natürlichem Nervengewebe zeigt.

Aufgrund der Beobachtung, dass das erfindungsgemäße Nervenimplantat zumindest in einigen Nerven bzw. Implantatempfängern in der Lage ist, über Fehlstellen von Nerven hinweg bereits ohne Wachstum von Nervenzellen eines Implantatempfängers eine zumindest teilweise Reizleitung zu ermöglichen, wird derzeit angenommen, dass die in dem Nervenimplantat enthaltenen Fäden der Spinnenseide nach der Implantation in die Unterbrechung bzw. Fehlstelle eines Nervs in der Lage sind, elektrische Nervenimpulse zumindest teilweise zu leiten.

Ein besonderer Vorteil der erfindungsgemäßen Nervenimplantate liegt darin, dass die Regeneration von Nerven über ihre Unterbrechung bzw. Fehlstelle hinweg innerhalb kurzer Zeiträume erfolgt. Als Konsequenz dieser schnellen Regeneration unterbrochener Nerven, lässt sich feststellen, dass keine signifikante histologisch nachweisbare Degeneration des die Unterbrechung bzw. Fehlstelle des Nervs umgebenden Gewebes nachweisbar ist.

Ein besonderer Vorteil der erfindungsgemäßen Nervenimplantate liegt darin, dass innerhalb einiger Tage bis weniger Wochen die mit einem erfindungsgemäßen Nervenimplantat verbundenen Enden, die eine Unterbrechung bzw. Fehlstelle von Nerven begrenzen, in dem Bereich des Nervenimplantats histologisch nicht vom natürlichen Nervengewebe unterscheidbares Gewebe aufweisen und die Fehlstelle vorzugsweise auch von Schwann-Zellen überbrückt wird.

So wird nach der Implantation erfindungsgemäßer Nervenimplantate in Fehlstellen von motorischen Nerven beobachtet, dass durch einen Unfall oder eine Operation abgetrennte Axone durch neu aussprossende Axone ersetzt werden und über das Nervenimplantat hinweg eine schnelle Rekonstruktion des natürlichen Nervengewebes im Bereich der Fehlstelle stattfindet.

Ein weiterer Vorteil der erfindungsgemäßen Nervenimplantate liegt darin, dass die als wesentlich für ihre Funktion erachtete fadenförmige Spinnenseide von Spinnen, vorzugsweise unter sterilen Bedingungen gehalten, steril in ausreichender Menge entnommen werden kann und unmittelbar zur Verwendung in einem erfindungsgemäßen Nervenimplantat zur Verfügung steht.

Für die Zwecke der Erfindung wird unter dem Begriff Spinnenseide, bzw. unter Fäden aus Spinnenseide vorzugsweise die von Radnetzspinnen erhältliche Spinnenseide, beispielsweise von Spinnen der Gattung *Nephila,* insbesondere *Nephila clavipes* verstanden. Weiterhin, weniger bevorzugt, können für die Zwecke der Erfindung unter dem Begriff Spinnenseide modifizierte Spinnenseide, beispielsweise physikalisch durch Kühlen und/oder Erwärmen, Strecken oder Bestrahlen behandelte Spinnenseide, oder chemisch modifizierte Spinnenseide gefasst werden, sowie Fäden, die biotechnologisch aus in Mikroorganismen, Pflanzen oder Insekten hergestellten Spinnenseidenproteinen gewonnen werden. Weiterhin fallen unter den Begriff Spinnenseide für die Zwecke der Erfindung auch Proteinfäden, die von anderen Insekten hergestellt werden, wie beispielsweise von Schmetterlingsraupen, insbesondere Seidenraupen, oder Motten erhältliche Proteinfäden.

Falls erforderlich, kann die Spinnenseide zur erfindungsgemäßen Verwendung in einem Nervenimplantat sterilisiert werden, vorzugsweise durch γ-Bestrahlung.

### Genaue Beschreibung der Erfindung

Erfindungsgemäße Nervenimplantate werden zur Überbrückung von Unterbrechungen oder Fehlstellen in Nerven, darunter motorischen Nerven, wie beispielsweise der Ischiasnerv oder der Plexus brachialis, oder sensorischen Nerven, wie Sehnerv und Hörnerv, sowie in vegetativen Nerven eingesetzt. Diese Nervenimplantate sind insbesondere bei Säugetieren und bevorzugterveise beim Menschen einsetzbar.

Dazu werden in einem chirurgischen Eingriff erfindungsgemäße Nervenimplantate mit einem ersten Ende der darin enthaltenen fadenförmige Spinnenseide mit Nervenenden eines ersten Endes einer Unterbrechung oder Fehlstelle im Nerv verbunden, sowie ein zweites Ende der im Nervenimplantat enthaltenen fadenförmigen Spinnenseide mit dem gegenüberliegenden, zweiten Nervenende der Unterbrechung bzw. Fehlstelle des Nerven. Die Verbindung zwischen erfindungsgemäßem Nervenimplantat und den Nervenenden, die die Unterbrechung oder Fehlstelle begrenzen, erfolgt durch eine räumlich nahe Anordnung von erstem Nervenende und erstem Ende eines Nervenimplantats, d.h. der darin enthaltenen fadenförmigen Spinnenseide, sowie räumlich nahe Anordnung von zweitem Nervenende mit zweitem Ende des Nervenimplantat, d.h. der darin enthaltenen fadenförmigen Spinnenseide.

Die räumlich nahe Anordnung wird vorzugsweise durch Nähen, beispielsweise mit bekanntem chirurgischen Nahtmaterial, oder Kleben fixiert, beispielsweise mit bekanntem Fibrinkleber.

In den Ausführungsformen des erfindungsgemäßen Nervenimplantats, in denen fadenförmige Spinnenseide innerhalb einer Hülle, vorzugsweise einer autologen oder azellularisierten Venole angeordnet sind, ist es alternativ zur unmittelbaren räumlichen Anordnung, optional in Verbindung mit einer Fixierung der Spinnenseide an den Nervenenden auch möglich, die miteinander zu verbindenden, unterbrochenen Nervenenden abschnittsweise in das offene Ende der Hülle des Nervenimplantats einzuführen und dort zu fixieren; beispielsweise durch Anbinden oder Annähen bzw. Ankleben eines Nervenendes mit einem Ende der Hülle des Nervenimplantats.

In einer bevorzugten Ausführungsform ist die fadenförmige Spinnenseide in Form von einem, vorzugsweise 10 bis 10000, bevorzugter 100 bis 1000 parallelen Abschnitten von fadenförmiger Spinnenseide in dem Nervenimplantat enthalten.

Alternativ ist es jedoch auch möglich, die fadenförmige Spinnenseide innerhalb des Nervenimplantats in eine andere Struktur zu bringen, beispielsweise zu verdrillen, in mehreren Bündeln fadenförmige Spinnenseide umeinander zu legen, wie beispielsweise die Spinnenseide bündelweise miteinander zu verflechten, oder die fadenförmige Spinnenseide um ein Trägerelement herum ein- oder mehrschichtig, gleich- oder gegenläufig zu wickeln. Dabei kann ein erfindungsgemäßes Nervenimplantat entsprechend der zweiten Ausführungsform gestaltet sein, oder als Zusatz zur ersten Ausführungsform ein Trägerelement aufweisen, so dass dieses auch innerhalb der Hülle angeordnet ist. Als Trägerelement können dabei eine autologe oder azelluläre Venole oder ein stift- oder streifenförmiger Wandabschnitt einer solchen autologen oder azellulären Venole dienen.

Die Erfindung wird nun genauer mit Bezug auf die Figuren beschrieben, in denen
- Figur 1 eine schematische Darstellung der ersten Ausführungsform eines nicht erfindungsgemäßen Nervenimplantats zeigt,
- Figur 2 eine schematische Darstellung der ersten Ausführungsform eines erfindungsgemäßen Nervenimplantats zeigt,
- Figur 3 eine schematische Darstellung der zweiten Ausführungsform nicht erfindungsgemäßer Nervenimplantate zeigt,
- Figur 4 eine schematische Darstellung

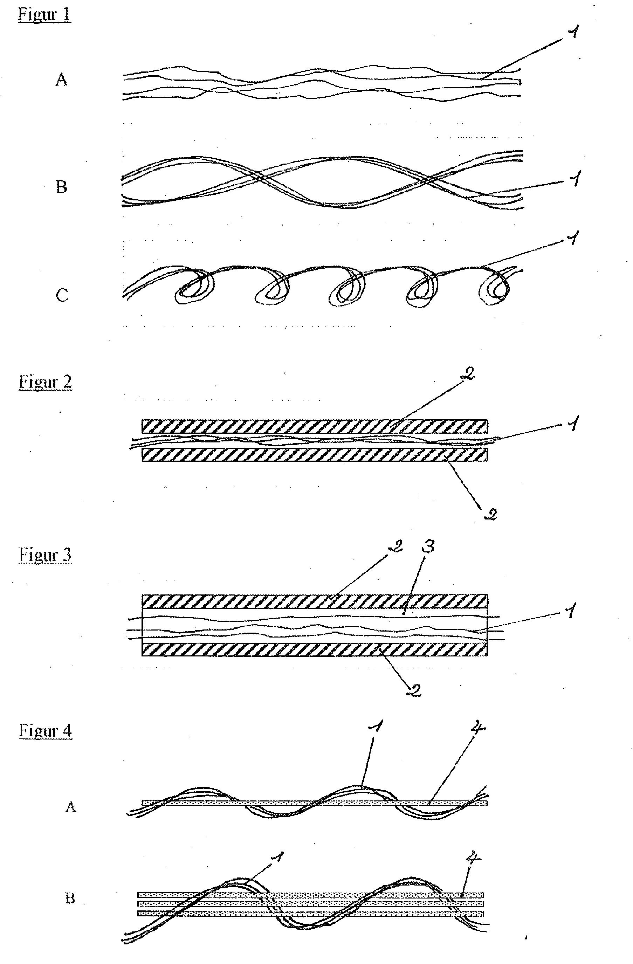

- Figur 4 eine schematische Darstellung der zweiten Ausführungsform erfindungsgemäßer Nervenimplantate zeigt.

In Figur 1 sind schematisch Anordnungen von fadenförmiger Spinnenseide in nicht erfindungsgemäßen Nervenimplantaten gezeigt, nämlich unter A) im wesentlichen parallel angeordnete Abschnitte von Spinnenseide 1, unter B) umeinander geschlungene Bündel von fadenförmiger Spinnenseide 1, sowie unter C) ein in sich verdrilltes Bündel von fadenförmiger Spinnenseide 1.

Es wird angenommen, dass die fadenförmige Spinnenseide als Richtschnur bzw. Leitbahnen für das Wachstum von Nervenzellen, insbesondere von Schwann-Zellen wirkt, so dass sich die Nervenzellen daran ausrichten und orientieren können, um die Unterbrechung bzw. die Defektstelle zwischen zwei getrennten Nervenenden zu überbrücken.

In Figur 2 ist die erste Ausführungsform erfindungsgemäßer Nervenimplantate schematisch im Schnitt dargestellt, in der fadenförmige Spinnenseide 1 innerhalb einer Hülle 2 angeordnet ist, vorzugsweise im Wesentlichen parallel zur Längsachse der Hülle 2.

In Figur 3 ist die zweite Ausführungsform nicht erfindungsgemäßer Nervenimplantate schematisch gezeigt, in denen fadenförmige Spinnenseide 1 im Wesentlichen in Längsrichtung innerhalb einer Hülle 2 angeordnet ist, wobei der verbleibende Zwischenraum zwischen Spinnenseide 1 und der inneren Oberfläche der Hülle 2 durch ein biokompatibles Matrixmaterial 3 ausgeführt wird, optional in Mischung mit autologen oder immunologisch kompatiblen kultivierten Nervenzellen, vorzugsweise Schwann-Zellen.

Figur 4 zeigt mögliche Ausführungen der zweiten Ausführungsform der erfindungsgemäßen Nervenimplantate, die ein Trägerelement 4 aufweist, um das herum fadenförmige Spinnenseide 1 angeordnet ist, beispielsweise in einem Bündel um das Trägerelement 4 herum gewickelt ist, oder in mehreren Bündel gleich- oder gegenläufig um das Trägerelement 4 herum gewickelt ist. Das Trägerelement 4 ist vorzugsweise ein Streifen, der aus einer autologen Venole genommen wurde, oder Streifen einer azellularisierten Venole,

### Beispiel 1: Erzeugung von Spinnenseide.

Als Beispiel für Spinnenseide, die zur Verwendung in einem erfindungsgemäßen Nervenimplantat geeignet ist, wurde ein Weibchen von *Nephila clavipes* mit Klebeband auf einer Unterlage fixiert. Nach mechanischer Reizung der Spinndrüse konnte der abgesonderte Faden aus Spinnenseide gewonnen werden, die für die Zwecke von Versuchen in einer clean bench um ein steriles 50 mL Falconröhrchen aufgewickelt wurden. Dazu konnte das Falconröhrchen mit einer Umdrehungszahl von 5 bis 20 /min rotieren gelassen werden.

Für die nachfolgenden Untersuchungen und Beispiele wurde solchermaßen gewonnene fadenförmige natürliche Spinnenseide ohne weitere Behandlung oder Sterilisation verwendet.

### Beispiel 2: Überbrückung einer Defektstelle im Ischiasnerv mit einer Distanz von 2,5 cm

Als Beispiel für einen motorischen Nerv mit einer Fehlstelle wurden Ratten ein Stück des Ischiasnervs im linken Hinterbein über eine Distanz von 2,5 cm chirurgisch entfernt. Während derselben Operation unter sterilen Bedingungen wurde die Fehlstelle bei unterschiedlichen Tieren von
1) einem Bündel von ca. 250 bis 350 annähernd parallel angeordneten Fäden von Spinnenseide, ca. 2,8 cm Länge, oder
2) zwei Bündeln zu je ca. 200 Fäden aus Spinnenseide, ca. 3 cm Länge, die um einen Streifen (1 mm Kantenlänge) azellulärisierter Venolen gegenläufig gewunden war,
verbunden. Die Spinnenseide wurde gemäß Beispiel 1 erzeugt.

Dabei wurden die Nervenenden des Ischiasnervs, die die Fehlstelle begrenzten, den Enden der Spinnenseide des Nervenimplantats gegenüber angeordnet und alternativ durch
a) Umwickeln der Kopf an Kopf stoßenden Enden mit Spinnenseide,
b) durch Vernähen mit chirurgischem Nahtmaterial oder
c) durch Verkleben mit Fibrinkleber aneinander fixiert.

Dabei wurde beobachtet, dass die Ratten nach dem Erwachen aus der Narkose die linke Hinterpfote nicht, wie sonst bei Unterbrechungen des Ischiasnervs üblich, in Form einer Kralle unnatürlich hielten, sondern vielmehr die Pfote in ihrer natürlichen offenen Haltung einsetzten. Ohne dass eine weitere Operation erforderlich gewesen wäre, konnten die operierten Tiere nach 2 bis 4 Tagen ihr linkes Hinterbein ohne signifikante Beeinträchtigung natürlich bewegen.

Bei einer histologischen Untersuchung des Nervenimplantats wurde fünf bis zehn Tagen nach der Implantation festgestellt, dass sich im Bereich der Unterbrechung des Nervs, die vom erfindungsgemäßen Nervenimplantat überbrückt worden war, Nervengewebe ausgebildet hatte, das keine signifikanten histologischen Abweichungen von natürlichem Nervengewebe aufwies.

Diese Nervenimplantate wurden auch zur Überbrückung von Defektstellen (1, 3 und 5 mm) des Hörnervs und des Sehnervs beim Kaninchen eingesetzt. Eine Reizleitung konnte innerhalb von 2 bis 5 oder 10 Tagen nach der Implantation beobachtet werden.

Bei den Nervenimplantaten, die gemäß 2) ein Trägerelement aufwiesen, wurde festgestellt, dass sich das azellularisierte Venolenstreifchen vollständig dem umliegenden Gewebe angepasst hatte.

Entzündliche Prozesse im Bereich der Spinnenseide wurden nicht beobachtet.

### Beispiel 3: Nervenimplantat mit Hülle

Als Beispiel für die zweite Ausführungsform erfindungsgemäßer Nervenimplantate wurden ca. 800- 1000 Spinnenseidefäden die gemäß Beispiel 1 erzeugt wurden, auf eine Länge von ca. 3,5 cm zugeschnitten und im wesentlichen axial in eine azellularisierte Venole eingezogen. Für die Zwecke dieser Erfindung kann eine Hülle als Bestandteil eines Nervenimplantats auch einen Längsschnitt aufweisen, so dass die Spinnenseide in eine längs aufgetrennte Hülle eingelegt werden können. Anschließend kann der Längsschnitt der Hülle geschlossen werden, z.B. ohne zusätzliche Mittel durch die Eigenspannung des Hüllmaterials durch Annähern der Schnittkanten des Längsschnitts, oder durch Vernähen oder Verkleben des Längsschnitts, oder durch Umwickeln der Hülle, beispielsweise mit einem Spinnenseidefaden oder chirurgischem Nahtmaterial.

Azellularisierte Venolen oder Streifen azellularisierter Venolen, die in den erfindungsgemäßen Nervenimplantaten verwendbar sind, wurden aus peripheren Venen hergestellt. Als Beispiel dafür wurden Beinvenen von Schweinen entnommen, die für andere experimentelle Zwecke getötet worden waren. Die Venolen hatten einen Durchmesser von ca. 2 bis 3 mm und einer Länge von 5 cm. Zur Azellularisierung wurden diese Venolen über 24 Stunden in eine Trypsinlösung (0,05 Gew.-% Trypsin, 0,02 Gew.-% EDTA) gelegt und unter Schütteln inkubiert. Die Trypsinlösung wurde anschließend entfernt und die nun zellfreien Venolen wurden durch Inkubation mit PBS unter Schütteln gereinigt, wobei diese Waschung mindestens einmal wiederholt wurde. Derart azellularisierte Venolen konnten unmittelbar verwendet oder zwischenzeitlich bei -80 °C tiefgefroren werden.

Zur Verbindung des Nervenimplantats mit den Nervenenden, die die Fehlstelle begrenzen, wurden die Nervenenden jeweils in ein Ende der Hülle eingeführt, wobei die darin angeordnete Spinnenseide so bemessen war, dass sie die Nervenenden kontaktierte, vorzugsweise Kopf an Kopf bzw. Ende gegenüber Ende. Zur Fixierung wurden die Nervenenden jeweils an einem Ende der Hülle angenäht.

Alternative Nervenimplantate dieser Ausführungsform wiesen eine Länge der Spinnenseidefäden auf, die über die Enden der Hülle hinausragte, z.B. um ca. 3 - 5 mm an jedem Ende. Dann wurden die Nervenenden dadurch mit dem Nervenimplantat verbunden, dass die Abschnitte der Spinnenseidefäden, die sich außerhalb der Hülle befanden, jeweils gegen ein Nervenende gehalten wurden und die aneinandergrenzenden Enden mit Spinnenseide umwickelt wurden, oder mit Fibrinkleber bestrichen wurden.

Bei der Verwendung dieser Nervenimplantate in Fehlstellen des Ischiasnervs der Ratte gemäß Beispiel 3 konnten Distanzen von ca. 5 cm überbrückt werden, so dass die Funktionalität des Nervs innerhalb von 3 bis 5 Tagen nach der Implantation wieder hergestellt war.

Entsprechend Beispiel 3 wurden auch hier keine zwischenzeitliche krallenartige Haltung der betreffenden Pfote beobachtet. Die histologische Untersuchung ergab auch hier ca. 5 bis 10 Tage nach der Implantation keine signifikanten Abweichungen von natürlichem Nervengewebe.

## Patentansprüche

1. Nervenimplantat zur Überbrückung von Unterbrechungen oder Defektstellen innerhalb eines Nervs, **dadurch gekennzeichnet, dass** das Nervenimplantat aus fadenförmiger Spinnenseide (1) gebildet ist, die axialer Richtung innerhalb einer Hülle (2) oder in Längsrichtung ausgerichtet um ein Trägerelement (4) angeordnet ist, wobei die Hülle (2) eine autologe Venole, eine xenologe azellularisierte Venole oder eine immunologisch kompatible Venole ist, und das Trägerelement (4) eine autologe oder azellularisierte Venole, oder streifenförmige Elemente aus der Wandung autologer oder azellularisierter Venolen ist.

2. Nervenimplantat nach Anspruch 1, **dadurch gekennzeichnet, dass** das Nervenimplantat aus fadenförmiger Spinnenseide besteht.

3. Nervenimplantat nach Anspruch 1 oder 2, **dadurch gekennzeichnet, dass** die fadenförmige Spinnenseide (1) natürliche Spinnenseide ist.

4. Nervenimplantat nach Anspruch 3, **dadurch gekennzeichnet, dass** die natürliche Spinnenseide von einer Radnetzspinne erhältlich ist.

## Claims

1. Neural implant for bridging interruptions or defects within a nerve, **characterized in that** the neural implant is formed of thread-like spider silk (1), which is arranged around a carrier element (4) and aligned in axial direction within a sheathing (2) or in longitudinal direction around a carrier element, wherein the sheathing (2) is an autologous venule, a xenologous acellularized venule or an immunologically compatible venule, and the carrier element (4) is an autologous or acellularized venule, or strip-shaped elements from the wall of autologous or acellularized venules.

2. Neural implant according to claim 1, **characterized in that** the neural implant consists of thread-like spider silk.

3. Neural implant according to claim 1 or 2, **characterized in that** the thread-like spider silk (1) is natural spider silk.

4. Neural implant according to claim 3, **characterized in that** the natural spider silk is obtainable from an orb-weaver spider.

## Revendications

1. Implant nerveux servant à ponter des interruptions ou des points défectueux d'un nerf, **caractérisé par le fait que** l'implant nerveux est formé d'une soie d'araignée (1) en forme de fil, qui est disposée axialement à l'intérieur d'une enveloppe (2) ou, orientée longitudinalement, autour d'un élément de support (4), l'enveloppe (2) est une veinule autologue, une veinule xénologue acellulaire ou une veinule à compatibilité immunologique, et l'élément de support (4) est une veinule autologue ou acellulaire ou des éléments en forme de bandes provenant de la paroi des veinules autologues ou acellulaires.

2. Implant nerveux selon la revendication 1, **caractérisé par le fait que** l'implant nerveux est constitué d'une soie d'araignée en forme de fil.

3. Implant nerveux selon la revendication 1 ou 2, **caractérisé par le fait que** la soie d'araignée (1) en forme de soie est une soie d'araignée naturelle.

4. Implant nerveux selon la revendication 3, **caractérisé par le fait que** la soie d'araignée provient d'une araignée de la famille des araneidae.
